# FASCICULE DE BREVET EUROPEEN

(11) **EP 4 453 463 B1**
(45) Date de publication et mention de la délivrance du brevet: **18.02.2026**
(21) Numéro de dépôt: 22843168.0
(22) Date de dépôt: 20.12.2022
(51) Int. Cl.: F16L 37/26, A61M 39/16, A61M 39/18

(54) **DISPOSITIF DE RACCORDEMENT ASEPTIQUE D'UN TUBE**
ASEPTISCHE KUPPLUNGSVORRICHTUNG FÜR EIN ROHR
ASEPTIC COUPLING DEVICE FOR A TUBE

(30) Priorité: 21.12.2021 FR 2114194
(43) Date de publication de la demande: 30.10.2024
(73) Titulaire: Parker Hannifin EMEA S.à.r.l., 1163 Etoy (CH)
(72) Inventeur: LE QUERE, Philippe, 35830 Betton (FR)
(74) Mandataire: Cabinet Boettcher
(86) Numéro de dépôt international: PCT/EP2022/086943
(87) Numéro de publication internationale: WO 2023/118108

(56) Documents cités:
- EP-A1- 3 225 895
- DE-A1- 102013 214 068
- US-A1- 2006 252 298

## Description

La présente invention concerne le domaine du transport de fluides liquides ou gazeux et plus particulièrement un dispositif de raccordement aseptique destiné à réaliser le raccordement de deux tubes stériles.

### ARRIERE PLAN DE L'INVENTION

On connaît des ensembles de raccordement destinés à connecter des canalisations stériles reliées à des éléments de circuits de l'industrie pharmaceutiques, tels que des filtres, des centrifugeuses, des pompes, des réservoirs en plastique souple ou des conteneurs de traitement comme des bio-processeurs.

Un tel ensemble comprend généralement deux dispositifs de raccordement destinés à être connectés entre eux après que chacun ait été raccordé à une extrémité de canalisation. Chaque dispositif comprend un corps tubulaire délimitant un canal agencé pour recevoir l'extrémité de canalisation. Le corps comporte une face frontale sur laquelle est fermement fixé un élément d'étanchéité annulaire avec lequel l'extrémité de canalisation est destinée à rentrer en contact étanche. L'élément d'étanchéité est recouvert d'un film de protection en forme de bande repliée sur elle-même dont une partie adhère de manière amovible à un flanc latéral libre de l'élément d'étanchéité et une autre partie forme une languette de tirage s'étendant en saillie de la première partie.

Après que les extrémités de canalisation aient été emmanchées dans les corps, les dispositifs sont tout d'abord mis coaxialement en regard puis rapprochés l'un de l'autre jusqu'à mettre en contact les films de protection et comprimer légèrement les éléments d'étanchéité. Le retrait simultané des films de protection par tirage des languettes entraîne alors une mise en contact étanche des flancs latéraux des éléments d'étanchéité entre eux et donc la formation d'un canal aseptique de passage de fluide entre les deux canalisations. Un rapprochement supplémentaire des dispositifs est ensuite réalisé avant de verrouiller les positions relatives des dispositifs au moyen d'une bride de serrage. Ces différentes étapes de mise en œuvre du raccordement sont fastidieuses et par ailleurs, l'effort de rapprochement supplémentaire peut, lorsqu'il est répété, engendrer des troubles musculosquelettiques.

Il est par ailleurs connu, notamment du document US-A-1365776, un dispositif de raccordement d'un tube, comprenant :
- un corps pourvu d'un embout tubulaire qui est agencé pour être engagé dans le tube, un canal s'étendant longitudinalement dans l'embout et débouchant dans un logement formé dans le corps et ouvert sur une surface du corps opposée à l'embout, le logement recevant un élément d'étanchéité annulaire ;
- un couvercle de protection de l'élément d'étanchéité, monté coulissant sur le corps parallèlement à ladite surface, entre une position d'obturation du logement et une position de dégagement du logement ; et
- des moyens de couplage à un autre dispositif de raccordement.

Le raccordement demande un effort de compression de l'élément d'étanchéité et les frottements de l'élément d'étanchéité sur le couvercle puis contre l'élément d'étanchéité de l'autre dispositif de raccordement lors du raccordement entre eux des deux dispositifs de raccordement obligent l'opérateur à des efforts importants pouvant engendrer des troubles musculosquelettiques.

### OBJET DE L'INVENTION

L'invention a donc pour but de proposer un dispositif de raccordement aseptique d'un tube permettant d'obvier au moins en partie aux inconvénients précités.

### RESUME DE L'INVENTION

A cet effet, l'invention propose un dispositif de raccordement aseptique d'un tube comprenant :
- un corps pourvu d'un embout tubulaire qui est agencé pour être engagé dans le tube, un canal s'étendant longitudinalement dans l'embout et débouchant dans un logement formé dans le corps et ouvert sur une surface dudit corps opposée à l'embout, le logement recevant un élément d'étanchéité annulaire ;
- un couvercle de protection monté coulissant sur le corps parallèlement à ladite surface, entre une position d'obturation du logement et une position de dégagement du logement ; et
- des moyens de couplage à un autre dispositif de raccordement.

Selon l'invention, l'élément d'étanchéité est monté mobile entre une première position en saillie de la surface du corps et une deuxième position en retrait de la première position, l'élément d'étanchéité étant rappelé dans ladite première position par des moyens de rappel élastique qui appliquent l'élément d'étanchéité contre le couvercle en position d'obturation.

Un tel agencement de l'élément d'étanchéité permet de limiter les efforts exercés par l'opérateur lors du raccordement entre eux de deux dispositifs de raccordement.

Selon un mode de réalisation particulier de l'invention, l'élément d'étanchéité comprend une collerette prenant appui contre un fond du logement pour former les moyens de rappel élastique.

De manière particulière, l'élément d'étanchéité est pourvu d'une armature.

Selon une caractéristique particulière, le dispositif comprend des moyens de verrouillage du couvercle dans chacune de ses deux positions.

Selon une autre caractéristique particulière, le couvercle est agencé pour se déplacer de la position d'obturation à la position de dégagement sous l'effet d'un effort transversal destiné à être exercé par un corps de l'autre dispositif de raccordement lors du raccordement entre eux des deux dispositifs de raccordement.

Selon une autre caractéristique particulière, les moyens de couplage comprennent un élément d'enfichage mâle et un élément d'enfichage femelle destinés à coopérer respectivement avec un élément d'enfichage femelle et un élément d'enfichage mâle de l'autre dispositif de raccordement.

De manière particulière, l'élément d'enfichage mâle et l'élément d'enfichage femelle s'étendent orthogonalement à l'embout.

L'invention concerne également un ensemble de raccordement de deux tubes comprenant deux tels dispositifs de raccordement.

Avantageusement, les deux dispositifs de raccordement sont identiques.

### BREVE DESCRIPTION DES DESSINS

L'invention sera mieux comprise à la lumière de la description qui suit, laquelle est purement illustrative et non limitative, et doit être lue en regard des dessins annexés, parmi lesquels :
[Fig. 1] la figure 1 est une vue en perspective d'un dispositif de raccordement selon un premier mode de réalisation de l'invention, dans laquelle le couvercle est dans une position d'obturation ;
[Fig. 2] la figure 2 est une vue analogue à celle de la figure 1, dans laquelle le couvercle est dans une position de dégagement ;
[Fig. 3] la figure 3 est une autre vue en perspective du dispositif de raccordement illustré à la figure 1 ;
[Fig. 4] la figure 4 est une vue en coupe axiale du dispositif de raccordement illustré à la figure 1 ;
[Fig. 5] la figure 5 est une vue de détail du dispositif de raccordement illustré à la figure 4, dans laquelle le couvercle est dans la position de dégagement ;
[Fig. 6A] la figure 6A est une vue en coupe axiale d'un ensemble de raccordement aseptique selon un mode de réalisation particulier de l'invention, avant connexion ;
[Fig. 6B] la figure 6B est une vue analogue à la figure 6A pendant la connexion, avant un déplacement des couvercles ;
[Fig. 6C] la figure 6C est une vue analogue à la figure 6A pendant la connexion, avant une mise en contact étanche des éléments d'étanchéité ;
[Fig. 6D] la figure 6D est une vue analogue à la figure 6A après connexion ;
[Fig. 7] la figure 7 est une vue analogue à la figure 5, illustrant une variante du dispositif de raccordement illustré à la figure 1 ;
[Fig. 8] la figure 8 est une vue en coupe axiale d'un dispositif de raccordement selon un deuxième mode de réalisation de l'invention ;
[Fig. 9] la figure 9 est une vue de détail du dispositif de raccordement illustré à la figure 8, dans laquelle le couvercle est dans la position de dégagement ;
[Fig. 10] la figure 10 est une vue analogue à la figure 9, illustrant une variante du dispositif de raccordement illustré à la figure 8.

### DESCRIPTION DETAILLEE DE L'INVENTION

L'invention est ici décrite en application au raccordement d'un premier tube 100 stérile formant un premier conduit de transport de fluide à un deuxième conduit de transport de fluide formé par un deuxième tube 200 stérile.

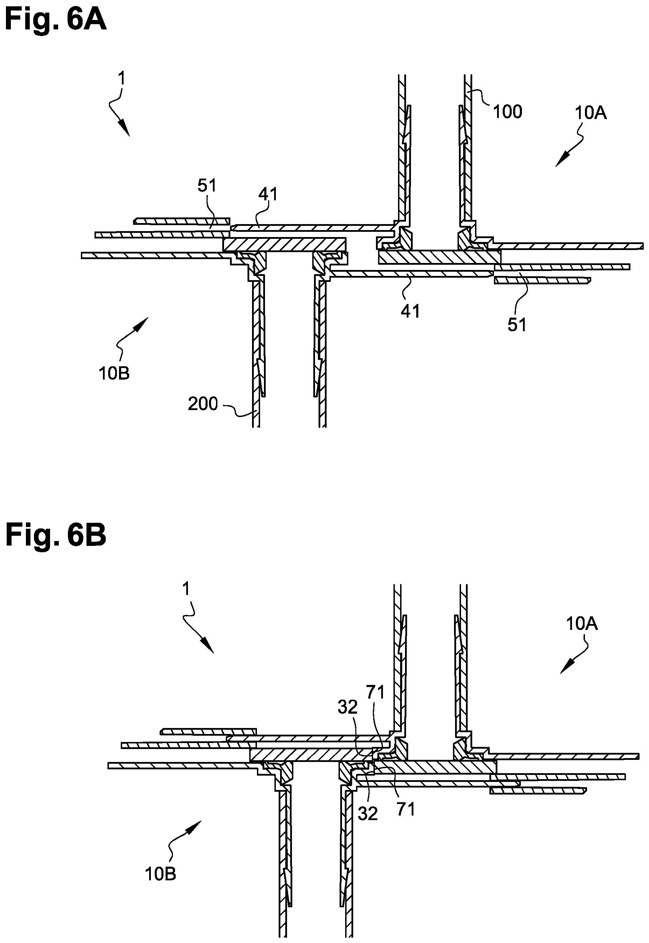

En référence aux figures 6A à 6D, un ensemble de raccordement fluidique généralement désigné en 1, selon un premier mode de réalisation de l'invention, comprend deux dispositifs 10 de raccordement destinés à réaliser ensemble une connexion aseptique entre le premier tube 100 et le deuxième tube 200. Les deux dispositifs 10 sont identiques et sont distingués sur les figures par l'adjonction des lettres A, B.

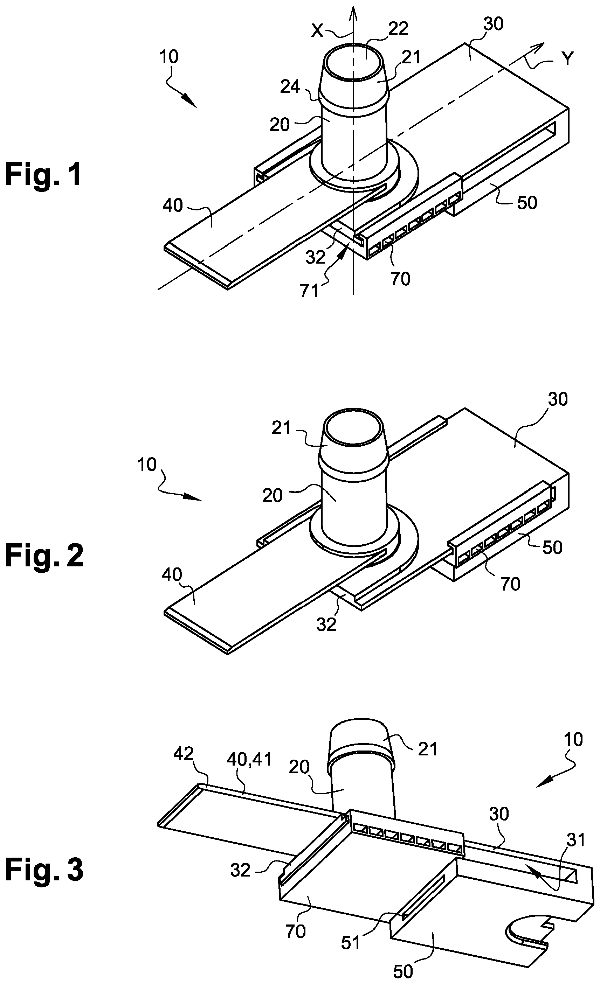

Comme illustré aux figures 1 à 4, chaque dispositif 10 comprend un corps 20 comportant un embout 21 tubulaire s'étendant selon un axe X. L'embout 21 délimite un canal 22 de passage de fluide débouchant dans un logement ouvert sur une face frontale 31 d'une plaque 30 rectangulaire s'étendant dans un plan orthogonal à l'axe X, selon un axe Y.

L'embout 21 comporte un premier tronçon d'extrémité délimitant un premier alésage 23.1 qui débouche sur une extrémité libre de l'embout 21 et qui forme une portion du canal 22. Le premier tronçon comprend extérieurement un relief 24 en dent de sapin et un premier épaulement 25.1 formant respectivement un moyen de son ancrage dans le tube 100, 200 et une butée à son enfoncement dans ledit tube 100, 200.

L'embout 21 comporte un deuxième tronçon d'extrémité délimitant un deuxième alésage 23.2 et un troisième alésage 23.3 tous deux coaxiaux au premier alésage 23.1 et formant ensemble le logement qui reçoit un élément d'étanchéité 60 annulaire. Le troisième alésage 23.3 débouche sur la face frontale 31 de la plaque 30 et a un diamètre supérieur à celui du deuxième alésage 23.2 qui a lui-même un diamètre supérieur à celui du premier alésage 23.1. Le deuxième tronçon comprend extérieurement un deuxième épaulement 25.2 agencé entre le premier épaulement 25.1 et la plaque 30.

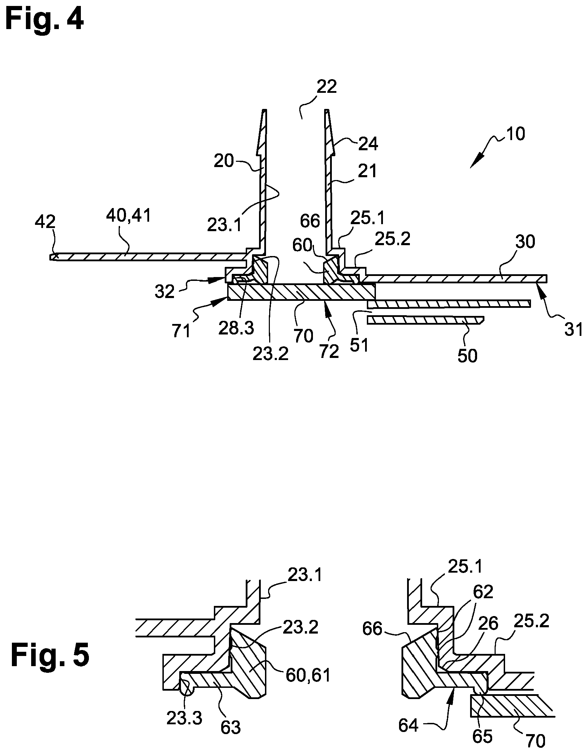

En référence à la figure 5, l'élément d'étanchéité 60 comprend un insert 61 tubulaire délimitant une section de passage de fluide globalement constante. L'insert 61 est pourvu extérieurement de deux reliefs 62 annulaire en contact étanche avec le deuxième alésage 23.2 et d'une collerette 63 dont un flanc latéral est en appui sur un fond du troisième alésage 23.3.

La collerette 63 est élastiquement déformable et définit une face frontale 64 de l'élément d'étanchéité 60 de laquelle s'étend en saille une première extrémité de l'insert 61 destinée à être en contact étanche avec celle de l'élément d'étanchéité 60 de l'autre dispositif 10 de raccordement. La collerette 63 a un pourtour extérieur comportant un bourrelet 65 annulaire s'étendant en saillie de la face frontale 64 et destiné à être en contact avec celui de l'autre dispositif 10 de raccordement.

L'insert 61 est monté mobile axialement dans le deuxième alésage 23.2 entre une première position dite « avancée » en saillie de la face frontale 31 de la plaque 30 et dans laquelle une deuxième extrémité de l'insert 61 est éloignée du fond du deuxième alésage 23.2 (figure 5), et une deuxième position dite « reculée » en retrait de la première position et dans laquelle la deuxième extrémité de l'insert 61 est en contact avec ledit fond du deuxième alésage 23.2 (figure 4). L'insert 61 est rappelé dans la position avancée par la collerette 63 passant d'un état déformé à un état de repos.

Dans la position avancée, l'insert 61 s'étend en saillie de la face frontale 31 de la plaque 30 d'une première hauteur. Dans la position reculée, l'insert 61 s'étend ici en saillie de la face frontale 31 de la plaque 30 d'une deuxième hauteur inférieure à la première hauteur. Cette deuxième hauteur peut aussi être nulle (l'insert 61 affleure la face frontale 31 de la plaque 30), voire négative (l'insert 61 est escamoté dans son logement).

On notera que la deuxième extrémité de l'insert 61 a ici une surface intérieure 66 de forme tronconique pour amener le fluide circulant dans le canal 22 à exercer sur ladite deuxième extrémité à exercer un effort tendant à amener l'insert 61 en position avancée. On notera également que le fond du troisième alésage 23.3 comprend une fraisure 26 agencée de manière à faciliter la déformation de la collerette 63 et son passage de l'état de repos à l'état déformé.

L'insert 61 est agencé pour être amené de sa position avancée à sa position reculée sous l'effet d'un effort axial exercé sur la première extrémité de l'insert 61 par un couvercle 70 de protection de forme globalement parallélépipédique et s'étendant dans un plan orthogonal à l'axe X. Le couvercle 70 est monté coulissant sur la plaque 30 parallèlement à la face frontale 31 de ladite plaque 30, entre une première position dite d'obturation du logement recevant l'élément d'étanchéité 60, dans laquelle il s'étend en regard dudit élément d'étanchéité 60 en étant en contact étanche avec le bourrelet 65 et en exerçant sur la première extrémité de l'insert 61 un effort axial de sorte que ledit insert 61 est en position reculée (figures 1 et 4), et une deuxième position dite de dégagement du logement recevant l'élément d'étanchéité, dans laquelle il s'étend en regard de la face frontale 31 de la plaque 30 de sorte que l'insert 61 est en position avancée (figures 2 et 5). L'élément d'étanchéité 60 est ainsi intégralement masqué par le couvercle 70 lorsque ce dernier est dans sa position d'obturation, et est parfaitement visible lorsque ledit couvercle 70 est dans sa position de dégagement.

Comme on le verra plus loin, le couvercle 70 est agencé pour être amené de sa position d'obturation à sa position de dégagement sous l'effet d'un effort transversal exercé sur une face avant 71 dudit couvercle 70 par une face avant 32 de la plaque 30 de l'autre dispositif 10.

Par ailleurs, le dispositif 10 comprend des moyens de verrouillage (non représentés) du couvercle 70 dans sa position d'obturation et dans sa position de dégagement.

Le corps 20 du dispositif 10 comprend également un élément d'enfichage mâle 40 et un élément d'enfichage femelle 50 destinés à coopérer respectivement avec l'élément d'enfichage femelle 50 et l'élément d'enfichage mâle 40 de l'autre dispositif 10 de raccordement.

L'élément d'enfichage mâle 40 comporte une languette 41 en forme de parallélépipède rectangle s'étendant depuis une surface externe de l'embout 21 délimitée par le premier épaulement 25.1 et le deuxième épaulement 25.2, dans un plan orthogonal à l'axe X suivant un axe parallèle à l'axe Y. La languette 41 a une largeur sensiblement égale à un diamètre du premier épaulement 25.1, et a une extrémité libre 42 doublement biseautée pour faciliter une insertion de l'élément d'enfichage mâle 40 dans l'élément d'enfichage femelle 50 de l'autre dispositif 10.

L'élément d'enfichage femelle 50 s'étend en regard de la face frontale 31 de la plaque 30 et comporte une fente 51 définissant un logement dans lequel est destiné à être inséré l'élément d'enfichage mâle 40 de l'autre dispositif 10. La fente 51 est agencée de manière à engendrer un coulissement dudit élément d'enfichage mâle 40 suivant un axe parallèle à l'axe Y tout au long de son insertion dans l'élément d'enfichage femelle 50.

Afin d'éviter toute détérioration et contamination de l'embout 21 lors du stockage et du transport du dispositif 10, un capot de protection (non représenté) est monté de manière amovible sur ledit embout 21. Le capot peut par exemple être agencé pour se clipper (par emboîtement élastique) sur le relief 24 en dent de sapin et est destiné à être retiré juste avant le couplage du dispositif 10 à l'autre dispositif 10.

On notera que l'ensemble du dispositif 10 est stérilisé en usine afin d'être exempt de toute contamination avant son stockage.

Le fonctionnement de l'ensemble de raccordement 1 va maintenant être détaillé en regard des figures 6A à 6D.

Le capot de protection du premier dispositif 10A est tout d'abord retiré de l'embout 21 rendant notamment visible le relief 24 en dent de sapin et le premier épaulement 25.1. L'embout 21 du premier dispositif 10A est alors emmanché dans le premier tube 100 jusqu'à ce que celui-ci vienne en butée contre le premier épaulement 25.1 dudit premier dispositif 10A. De même, le capot de protection du deuxième dispositif 10B est retiré de l'embout 21 qui est alors emmanché dans le deuxième tube 200 jusqu'à ce que celui-ci vienne en butée contre le premier épaulement 25.1 dudit deuxième dispositif 10B, de sorte que les premier et deuxième dispositifs 10A, 10B sont respectivement ancrés dans les premier et deuxième tubes 100, 200.

Les premier et deuxième dispositifs 10A, 10B sont ensuite présentés en regard l'un de l'autre, le premier dispositif 10A étant tourné axialement de 180 degrés par rapport au deuxième dispositif 10B de sorte que la languette 41 et la fente 51 du premier dispositif 10A sont respectivement en regard de la fente 51 et de la languette 41 du deuxième dispositif 10B (figure 6A).

Dans un premier temps, les premier et deuxième dispositifs 10A, 10B sont rapprochés transversalement l'un de l'autre, selon l'axe Y, jusqu'à ce que la face avant 32 des plaques 30 des premier et deuxième dispositifs 10A, 10B soit en appui contre la face avant 71 des couvercles 70 desdits premier et deuxième dispositifs 10A, 10B, la fente 51 du premier dispositif 10A recevant alors une portion de la languette 41 du deuxième dispositif 10B et la fente 51 du deuxième dispositif 10B recevant une portion de la languette 41 du premier dispositif 10A (figure 6B).

En poursuivant le rapprochement des premier et deuxième dispositifs 10A, 10B, la plaque 30 du premier dispositif 10A exerce sur le couvercle 70 du deuxième dispositif 10B un effort transversal entraînant un déplacement dudit couvercle 70 de sa position d'obturation vers sa position de dégagement. De même, la plaque 30 du deuxième dispositif 10B exerce sur le couvercle 70 du premier dispositif 10A un effort transversal entraînement un déplacement dudit couvercle 70 de sa position d'obturation vers sa position de dégagement.

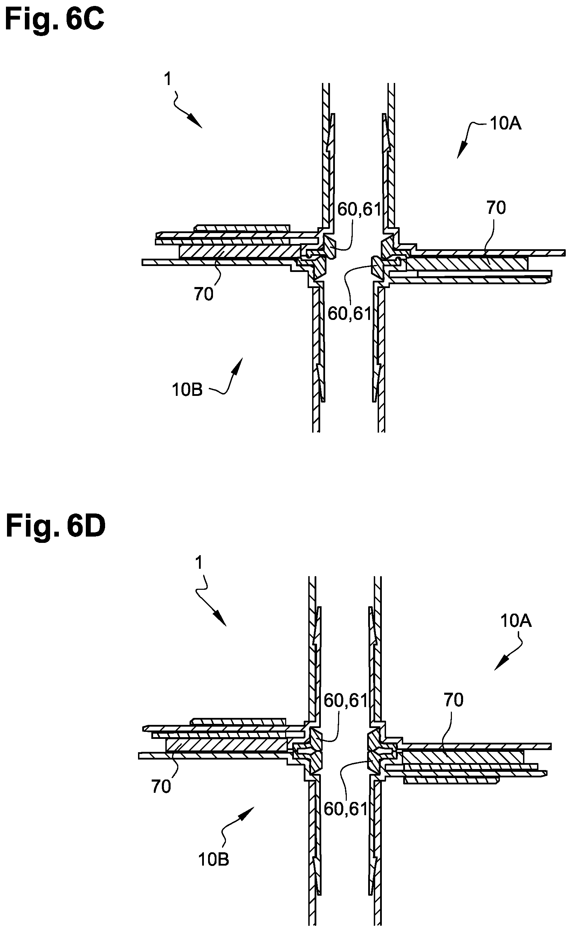

Dans un deuxième temps, les premier et deuxième dispositifs 10A, 10B continuent d'être rapprochés l'un de l'autre jusqu'à ce que les premières extrémités des inserts 61 des premier et deuxième dispositifs 10A, 10B soient en contact l'une de l'autre (figure 6C). En ne masquant plus les éléments d'étanchéité 60, les couvercles 70 n'exercent alors plus aucun effort sur les inserts 61 qui passent alors de la position reculée à la position avancée.

En poursuivant le rapprochement des premier et deuxième dispositifs 10A, 10B jusqu'à ce que l'embout 21 du premier du dispositif 10A s'étende coaxialement à l'embout 21 du deuxième dispositif 10B, les inserts 61 des premier et deuxième dispositifs 10A, 10B exercent l'un sur l'autre un effort axial entraînant le retour desdits inserts 61 en position reculée et assurant une mise en contact étanche de ceux-ci (figure 6D). Le bourrelet 65 du premier dispositif 10A est alors en contact étanche avec le bourrelet 65 du deuxième dispositif 10B et les extensions 73 des premier et deuxième dispositifs 10A, 10B coopèrent avec les encoches 54 des premier et deuxième dispositifs 10A, 10B.

On parle alors de couplage des premier et deuxième dispositifs 10A, 10B qui forment ainsi ensemble un canal pour le passage d'un fluide entre le premier tube 100 et le deuxième tube 200.

On notera que la pression du fluide circulant entre le premier tube 100 et le deuxième tube 200 exercera un effort axial sur les deuxièmes extrémités des inserts 61, ce qui aura pour effet d'augmenter la pression de contact entre les premières extrémités desdits inserts 61 formant pistons.

On notera également que le passage des inserts 61 de la position avancée à la position reculée à la fin du rapprochement des premier et deuxième dispositifs 10A, 10B permet de limiter les efforts exercés par l'opérateur pour rapprocher lesdits premier et deuxième dispositifs 10A, 10B.

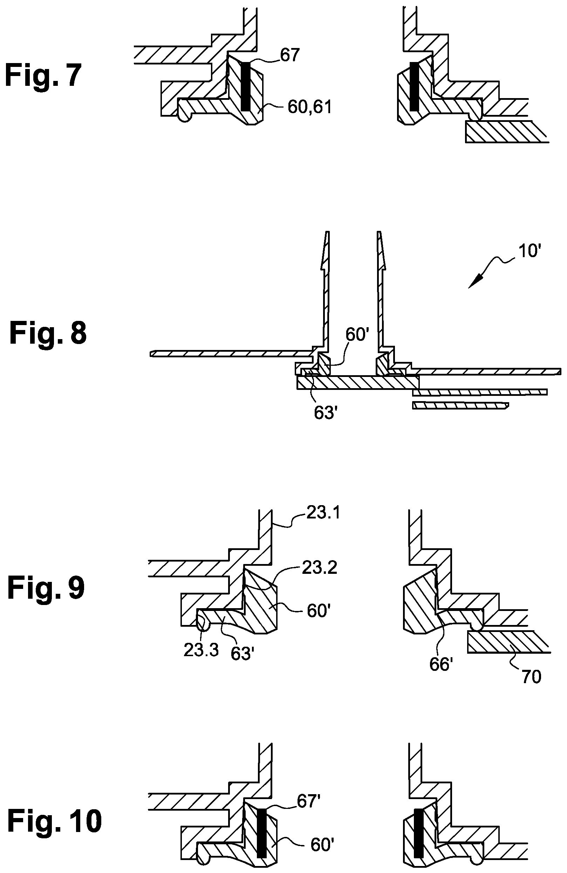

La figure 7 illustre une variante du dispositif de raccordement 10, dans laquelle l'insert 61 est pourvu d'une armature 67 permettant de limiter la déformation dudit insert 61 et donc son poumonage sous la pression du fluide. L'armature 67 est ici réalisée sous forme d'une portion tubulaire rigide/tissée s'étendant coaxialement à l'insert 61. L'armature peut être réalisé en matériau métallique, plastique (polymère)...

La figure 8 illustre un dispositif 10' de raccordement selon un deuxième mode de réalisation de l'invention. Le dispositif 10' diffère du dispositif 10 en ce qu'il comprend un élément d'étanchéité 60' ayant une collerette 63' de forme sensiblement différente de celle de la collerette 63 de l'élément d'étanchéité 60. En référence à la figure 9, un pourtour intérieur de la collerette 63' est pourvu d'une gorge annulaire 66' agencée de manière faciliter la déformation de la collerette 63' lors de son passage de l'état de repos à l'état déformé, en lieu et place de la fraisure 26 du troisième alésage 23.3 du dispositif 10.

La figure 10 illustre une variante du dispositif de raccordement 10', dans laquelle l'insert 61' est pourvu d'une armature métallique 67' permettant de limiter la déformation dudit insert 61 et donc son poumonage sous la pression du fluide. L'armature 67' est ici réalisée sous forme d'une portion tubulaire rigide/tissée s'étendant coaxialement à l'insert 61. L'armature peut être réalisé en matériau métallique, plastique (polymère)...

Bien entendu, l'invention n'est pas limitée au mode de réalisation décrit mais englobe toute variante entrant dans le champ de l'invention telle que définie par les revendications.

Bien que l'insert 61 de l'élément d'étanchéité 60 soit ici rappelé de sa position reculée à sa position avancée via la collerette 63, tout autre moyen de rappel élastique peut être utilisé (ressort en appui contre un fond du premier alésage 23.1 et sur la deuxième extrémité de l'insert, lames élastiques d'étendant en saille de la deuxième extrémité de l'insert et en appui contre un fond du premier alésage 23.1...).

Bien que l'ensemble de raccordement comprenne ici deux dispositifs 10A, 10B identiques, il peut aussi comprendre deux dispositifs différents.

Bien que l'ancrage des tubes 100, 200 sur les dispositifs 10A, 10B soit ici réalisé par un relief en dent de sapin, il peut aussi être réalisé par tout autre moyen tel qu'un jonc, une rondelle extérieurement dentée ou des bras élastiquement déformables.

Bien que la surface intérieure 66 de la deuxième extrémité de l'insert 61 soit ici de forme tronconique, elle peut être de forme radiale ou de tout autre forme permettant d'amener le fluide circulant dans le canal 22 à exercer sur ladite extrémité à exercer un effort tendant à amener l'insert 61 en position avancée.

Les armatures 67, 67' équipant les insert 61, 61' peuvent être surmoulées, collées, montées libre...

## Revendications

1. Dispositif (10, 10') de raccordement aseptique d'un tube (100, 200), comprenant :
- un corps (20) pourvu d'un embout (21) tubulaire qui est agencé pour être engagé dans le tube, un canal s'étendant longitudinalement dans l'embout et débouchant dans un logement formé dans le corps et ouvert sur une surface dudit corps opposée à l'embout, le logement recevant un élément d'étanchéité (60, 60') annulaire ;
- un couvercle (70) de protection monté coulissant sur le corps parallèlement à ladite surface, entre une position d'obturation du logement et une position de dégagement du logement ; et
- des moyens de couplage (40, 50) à un autre dispositif de raccordement ;
**caractérisé en ce que** l'élément d'étanchéité est monté mobile entre une première position en saillie de la surface du corps et une deuxième position en retrait de la première position, l'élément d'étanchéité étant rappelé dans ladite première position par des moyens de rappel élastique (63, 63'), et **en ce que** les moyens de rappel élastique appliquent l'élément d'étanchéité contre le couvercle en position d'obturation.

2. Dispositif (10, 10') de raccordement selon la revendication 1, dans lequel l'élément d'étanchéité (60, 60') comprend une collerette (63, 63') prenant appui contre un fond du logement pour former les moyens de rappel élastique.

3. Dispositif (10) de raccordement selon l'une quelconque des revendications précédentes, dans lequel l'élément d'étanchéité (60, 60') est pourvu d'une armature (67, 67').

4. Dispositif (10, 10') de raccordement selon l'une quelconque des revendications précédentes, comprenant des moyens de verrouillage du couvercle (70) dans chacune de ses deux positions.

5. Dispositif (10, 10') de raccordement selon l'une quelconque des revendications précédentes, dans lequel le couvercle (70) est agencé pour se déplacer de la position d'obturation à la position de dégagement sous l'effet d'un effort transversal destiné à être exercé par un corps de l'autre dispositif de raccordement lors du raccordement entre eux des deux dispositifs de raccordement.

6. Dispositif (10, 10') de raccordement selon l'une quelconque des revendications précédentes, dans lequel les moyens de couplage comprennent un élément d'enfichage mâle (40) et un élément d'enfichage femelle (50) destinés à coopérer respectivement avec un élément d'enfichage femelle et un élément d'enfichage mâle de l'autre dispositif de raccordement.

7. Dispositif (10, 10') de raccordement selon l'une quelconque des revendications précédentes, dans lequel l'élément d'enfichage mâle (40) et l'élément d'enfichage femelle (50) s'étendent orthogonalement à l'embout (21).

8. Ensemble (1) de raccordement de deux tubes (100, 200) comprenant deux dispositifs (10A, 10B) de raccordement selon l'une quelconque des revendications précédentes.

9. Ensemble (1) selon la revendication 8, dans lequel les deux dispositifs (10A, 10B) de raccordement sont identiques.

## Patentansprüche

1. Kupplungsvorrichtung (10, 10') zur aseptischen Verbindung eines Rohres (100, 200), wobei die Vorrichtung umfasst:
- einen Körper (20) mit einem rohrförmigen Endstück (21), das zum Einführen in das Rohr ausgelegt ist, wobei sich ein Kanal in Längsrichtung durch das Endstück hindurch erstreckt und in eine in dem Körper ausgebildete Aufnahme mündet, die zu einer dem Endstück entgegengesetzten Fläche des Körpers hin offen ist, wobei die Aufnahme ein ringförmiges Dichtungselement (60, 60') aufnimmt;
- eine Schutzabdeckung (70), die parallel zu dieser Fläche zwischen einer Abdeckstellung zum Verschließen der Aufnahme und einer Freigabestellung zur Freilegung der Aufnahme verschiebbar auf dem Körper angebracht ist; und
- Kupplungsmittel (40, 50) zum Koppeln mit einer anderen Kupplungsvorrichtung;
**dadurch gekennzeichnet, dass** das Dichtungselement beweglich zwischen einer aus der Körperoberfläche vorstehenden ersten Stellung und einer in Bezug auf die erste Stellung zurückversetzten zweiten Stellung angebracht ist, wobei das Dichtungselement von elastischen Rückstellmitteln (63, 63') in die erste Stellung zurückgestellt wird, und dass die elastischen Rückstellmittel das Dichtungselement gegen die in der Abdeckstellung befindliche Abdeckung andrücken.

2. Kupplungsvorrichtung (10, 10') nach Anspruch 1, wobei das Dichtungselement (60, 60') einen Kragen (63, 63') umfasst, der sich zur Bildung der elastischen Rückstellmittel gegen einen Boden der Aufnahme abstützt.

3. Kupplungsvorrichtung (10) nach einem der vorhergehenden Ansprüche, wobei das Dichtungselement (60, 60') mit einer Armierung (67, 67') ausgestattet ist.

4. Kupplungsvorrichtung (10, 10') nach einem der vorhergehenden Ansprüche, umfassend Verriegelungsmittel (70) zum Verriegeln der Abdeckung in jeder ihrer beiden Stellungen.

5. Kupplungsvorrichtung (10, 10') nach einem der vorhergehenden Ansprüche, wobei die Abdeckung (70) derart ausgelegt ist, dass sie unter Einwirkung einer von einem Körper der anderen Kupplungsvorrichtung auszuübenden Querkraft beim Verbinden beider Kupplungsvorrichtungen aus der Abdeckstellung in die Freigabestellung überführt wird.

6. Kupplungsvorrichtung (10, 10') nach einem der vorhergehenden Ansprüche, wobei die Kupplungsmittel ein männliches Steckelement (40) und ein weibliches Steckelement (50) umfassen, welche dazu bestimmt sind, jeweils mit einem weiblichen Steckelement und einem männlichen Steckelement der anderen Kupplungsvorrichtung zusammenzuwirken.

7. Kupplungsvorrichtung (10, 10') nach einem der vorhergehenden Ansprüche, wobei sich das männliche Steckelement (40) und das weibliche Steckelement (50) orthogonal zu dem Endstück (21) erstrecken.

8. Kupplungsanordnung (1) zur Verbindung zweier Rohre (100, 200), umfassend zwei Kupplungsvorrichtungen (10A, 10B) nach einem der vorhergehenden Ansprüche.

9. Anordnung (1) nach Anspruch 8, wobei die zwei Kupplungsvorrichtungen (10A, 10B) identisch sind.

## Claims

1. A device (10, 10') for the aseptic connection of a tube (100, 200), the device comprising:
- a body (20) provided with a tubular end piece (21) that is arranged to be engaged in the tube, a channel extending longitudinally in the end piece and opening into a housing formed in the body and open on a surface of said body opposite the end piece, the housing receiving an annular sealing element (60, 60');
- a protective cover (70) slidably mounted on the body parallel to said surface, between a housing closing position and a housing releasing position; and
- means (40, 50) for coupling to another connecting device;
**characterised in that** the sealing element is mounted so that it can move between a first position projecting from the surface of the body and a second position retracted from the first position, the sealing element being returned to said first position by elastic return means (63, 63'), and **in that** the elastic return means press the sealing element against the cover in the closing position.

2. The connecting device (10, 10') according to claim 1, wherein the sealing element (60, 60') comprises a collar (63, 63') bearing against a bottom of the housing to form the elastic return means.

3. The connecting device (10) according to any one of the preceding claims, wherein the sealing member (60, 60') is provided with a reinforcement (67, 67').

4. The connecting device (10, 10') according to any one of the preceding claims, comprising means for locking the cover (70) in each of its two positions.

5. The connecting device (10, 10') according to any one of the preceding claims, wherein the cover (70) is arranged to move from the closing position to the release position under the effect of a transverse force intended to be exerted by a body of the other connecting device when the two connecting devices are connected together.

6. The connecting device (10, 10') according to any one of the preceding claims, wherein the coupling means comprise a male plug-in element (40) and a female plug-in element (50) intended to cooperate respectively with a female plug-in element and a male plug-in element of the other connecting device.

7. The connecting device (10, 10') according to any one of the preceding claims, wherein the male plug-in element (40) and the female plug-in element (50) extend orthogonally to the tubular end piece (21).

8. A connecting assembly (1) for connecting together two tubes (100, 200), the connecting assembly comprising two connecting devices (10A, 10B) according to any one of the preceding claims.

9. The assembly (1) according to claim 8, wherein the two connecting devices (10A, 10B) are identical.
